(19) Europäisches Patentamt
European Patent Office
Office européen des brevets

(11) Numéro de publication: **0 206 904 B1**

(12) **FASCICULE DE BREVET EUROPEEN**

(45) Date de publication de fascicule du brevet: **18.03.92**

(51) Int. Cl.[5]: **C12P 13/04**, C12N 11/00, C12N 9/06, C12N 9/04

(21) Numéro de dépôt: **86401267.9**

(22) Date de dépôt: **11.06.86**

(54) **Procédé de production enzymatique de L-alpha-aminoacides à partir d'chi cétoacides.**

(30) Priorité: **13.06.85 FR 8509121**

(43) Date de publication de la demande:
**30.12.86 Bulletin 86/52**

(45) Mention de la délivrance du brevet:
**18.03.92 Bulletin 92/12**

(84) Etats contractants désignés:
**AT BE CH DE FR GB IT LI LU NL SE**

(56) Documents cités:
**EP-A- 0 023 346**
**EP-A- 0 095 950**
**EP-A- 0 132 999**
**EP-A- 0 167 058**
**EP-A- 0 206 460**
**FR-A- 2 217 296**
**US-A- 3 036 958**

**ARTIFICIAL ORGANS, vol. 3, no. 1, février 1979, pages 38-41; T.M.S. CHANG et al.: "Effects of glucose dehydrogenase in converting urea and ammonia into amino acid using artificial cells"**

**BIOCHIM. ET BIOPHYS. ACTA, vol. 36, 1959, pags 288-289; M.M. HONG et al.: "Distribution of L-alanine dehydrogenase and L-glutamate dehydrogenase in Bacilli"**

(73) Titulaire: **INSTITUT FRANCAIS DU PETROLE**
**4, Avenue de Bois Préau**
**F-92502 Rueil-Malmaison(FR)**

(72) Inventeur: **Ballerini, Daniel**
**103, rue du Pontel**
**F-78100 St Germain en Laye(FR)**
Inventeur: **Benoit, Yves**
**2 Mail Renaissance**
**F-95120 Ermont(FR)**
Inventeur: **Lemal, Jeannine**
**3, Chemin de Paradis**
**F-92500 Rueil-Malmaison(FR)**
Inventeur: **Monot, Frédéric**
**9 Parc du Belloy**
**F-78600 Le Mesnil le Roi(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

**PATENT ABSTRACTS OF JAPAN**, vol. 8, no. 122 (C-227)[1559], 8 juin 1984; & JP-A-59 34 890 (DAIICHI KAGAKU YAKUHIN K.K.) 25-02-1984

**J. GEN. MICROBIOL.**, vol. 17, 1957, pages 620-624; P.C. SHAH et al.: "Optical activity of amino acids formed by reductive amination in Bacillus subtilis"

**J. AM. CHEM. SOC.**, vol. 107, 1985, pages 4028-4031, American Chemical Society; C.-H. WONG et al.: "Enzymatic vs. fermentative synthesis: thermostable glucose dehydrogenase catalyzed regeneration of NAD(P)H for use in enzymatic synthesis"

## Description

La présente invention concerne un procédé de préparation de L-α aminoacides, et plus particulièrement, un procédé pour produire par voie enzymatique des L-α aminoacides par réduction enzymatique asymétrique des α-cétoacides correspondants. Elle concerne aussi les produits obtenus par le procédé.

Il est connu qu'une réaction de préparation enzymatique des L-α aminoacides est représentée par le schéma suivant :

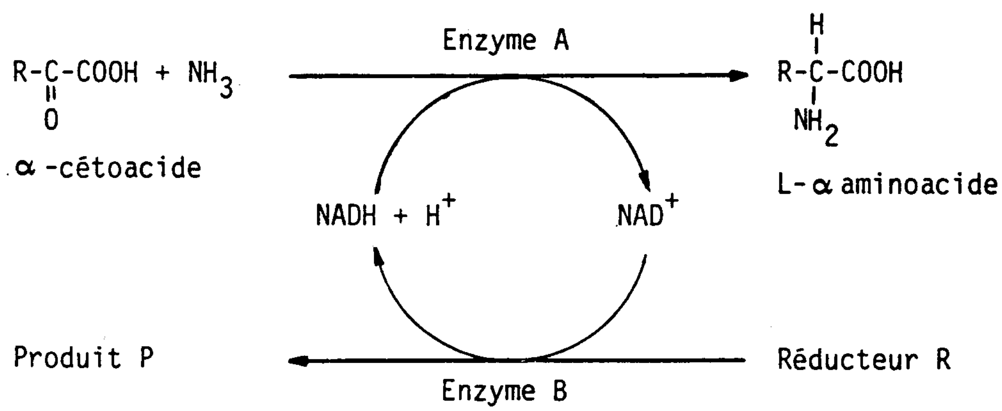


L'enzyme A, qui est une L-aminoacide deshydrogénase, catalyse la réduction spécifique de l'α-cétoacide en L-α aminoacide correspondant en présence indispensable d'un coenzyme qui peut avantageusement être le nicotinamide adénine dinucléotide. Le nicotinamide adénine dinucléotide réduit (NADH) est, dans cette réaction, oxydé à l'état de nicotinamide adénine dinucléotide ($NAD^+$). Il est très avantageux de le ramener sous forme de NADH dans une deuxième réaction et donc de le régénérer par un réducteur R qui est oxydé en un produit P, ladite deuxième réaction étant catalysée par l'enzyme B. Le réducteur peut être par exemple le glucose qui est oxydé par une glucose deshydrogénase en gluconolactone qui s'hydrolyse spontanément en acide gluconique.

Les enzymes A et B ainsi que le coenzyme sont employés de préférence en quantité très faible. Le réducteur R est un réactif et peut être présent en quantité substantielle, de préférence en quantité au moins stoechiométrique par rapport à l'α-cétoacide.

L'avantage d'un tel système est évident puisque le nicotinamide adénine dinucléotide qui est un produit coûteux, sous forme oxydée ou réduite, peut n'être utilisé qu'en quantité catalytique très faible puisqu'il est régénéré.

Il a été décrit dans le brevet US 3 036 958 l'utilisation notamment de Bacillus et particulièrement de Bacillus megaterium pour synthétiser du L-tryptophane à partir d'acide 3-indolepyruvique en présence d'un réactif donneur d'hydrogène et particulièrement le glucose. Mais ce document ne suggère, ni ne décrit l'utilisation d'un coenzyme $NADH/NAD^+$ que l'on régénèrerait en présence de glucose sous l'action d'un enzyme spécifique au cours d'une réaction secondaire.

De plus, le document Hong M.M. et al : Distribution of L-alanine dehydrogenase and L-glutamate dehydrogenase in bacilli" Biochem. et Biophys. Acta 36, 288-9 (1959) décrit que certaines souches de Bacillus peuvent avoir une activité L-alanine dehydrogènase et une activité L-glutamate deshydrogènase, ces deux activités enzymatiques étant $NAD^+$ dépendantes, mais ne précise en aucun cas que les souches de Bacillus possèdent l'activité glucose deshydrogènase nécessaire à la régénération du coenzyme.

Il a déjà été décrit dans le brevet FR 2 526 442 que la souche Bacillus megaterium ATCC 39118 (IFP 188) a la propriété de posséder une activité glucose deshydrogénase élevée (EC 1.1.1.47), qui conduit à la régénération de manière très avantageuse du NADH à partir de $NAD^+$ avec un système où le réducteur R est le glucose et le produit P l'acide gluconique après hydrolyse.

Par ailleurs, le brevet américain n° 4304858 décrit un procédé enzymatique continu du type précité selon lequel on synthétise des L-α aminoacides à partir d'α-cétoacides dans un réacteur à membrane avec le coenzyme nicotinamide adénine dinucléotide sous forme de dérivé à haut poids moléculaire soluble en milieu aqueux et avec deux enzymes purifiées, une deshydrogénase spécifique du substrat (L α-aminoacide deshydrogénase) et une formiate deshydrogénase utilisée pour la régénération du coenzyme.

Ce procédé comporte plusieurs inconvénients :

- On utilise deux enzymes intracellulaires provenant de deux microorganismes différents Candida

boidinii ou Pseudomonas oxalaticus pour la formiate deshydrogénase et Bacillus subtilis pour la L-aminoacide deshydrogénase, ce qui implique deux cultures différentes et deux procédures différentes d'extraction des enzymes intracellulaires :

- On emploie deux préparations enzymatiques purifiées, ce qui implique des opérations coûteuses de purification.

Un des objets de l'invention est de remédier à ces inconvénients. Un autre objet est de produire des L-α aminoacides tout en régénérant simultanément le nicotinamide adenine dinucléotide.

On propose donc un nouveau procédé de production de L-α aminoacide, dans lequel on soumet un mélange d'au moins un α-cétoacide ou d'au moins l'un de ses sels, d'un réducteur, d'une source d'ions ammonium et de nicotinamide adénine dinucléotide sous forme réduite ou oxydée à l'action d'une composition enzymatique unique ; ladite composition est le produit de culture d'une souche de Bacillus.

De préférence, on utilise le produit de culture d'une souche de Bacillus megaterium. On utilise de manière encore plus préférée le produit de culture de la souche de Bacillus megaterium ATCC 39118 en raison des rendements particulièrement élevés en L-α-aminoacides.

Cette souche est un mutant de sporulation de la souche de la collection du laboratoire Bacillus megaterium IFP 180 décrite dans le brevet FR 2 526 442.

Par ailleurs, le niveau de régénération de nicotinamide adenine dinucléotide est élevé puisqu'il peut être utilisé en quantité catalytique en général comprise entre 0,1 millimole par litre et 10 millimoles par litre.

On a découvert que la culture de souches de Bacillus, de manière préférée la culture de la souche de Bacillus megaterium et plus particulièrement de la souche ATCC 39 118 conduisent à une composition enzymatique présentant à la fois une activité L-aminoacide deshydrogènase, et une activité glucose deshydrogénase élevées, ce qui facilite la procédure de préparation et de purification des L-α aminoacides.

Dans le procédé de l'invention, on utilise généralement de préférence une composition enzymatique de Bacillus présentant les deux activités précitées en proportion de 0,1:1 a 10:1. De manière plus préférée, la composition enzymatique peut présenter des activités L-aminoacide deshydrogènase et glucose deshydrogènase sensiblement égales. On a notamment obtenu d'excellents rendements en L-α aminoacide en présence de la souche ATCC 39 118 de Bacillus megaterium dans ces conditions préférées.

Selon une caractéristique de l'invention, on peut travailler sur les extraits enzymatiques bruts, très peu purifiés ou purifiés de Bacillus, de Bacillus megaterium ou de la souche ATCC 39 118.

Les extraits bruts enzymatiques sont préparés par cassage des microorganismes récoltés par centrifugation ou filtration par exemple, d'une culture dont la croissance a été effectuée dans un milieu approprié. Pour le cassage des microorganismes, on utilise des techniques telles que le broyage avec des billes de verre, le traitement aux ultra-sons, etc. Après centrifugation des broyats de microorganismes pour éliminer les débris de parois cellulaires, on obtient l'extrait brut enzymatique.

Les extraits enzymatiques bruts, ainsi préparés, peuvent être utilisés pour la synthèse des L-aminoacides, en leur état ou sous forme immobilisée, dans des gels de polymères, par exemple de polyacrylamide, d'alginate ou de carrageenane par exemple.

On peut encore préparer des extraits enzymatiques purifiés à partir des extraits bruts après différents traitements tels que la précipitation au sulfate d'ammonium, un chauffage modéré par exemple. Les extraits enzymatiques purifiés sont alors utilisés, pour la synthèse des L-aminoacides, en leur état ou sous forme immobilisée.

Selon une autre caractéristique de l'invention on peut utiliser des cellules entières de Bacillus, de Bacillus megaterium ou de Bacillus megaterium ATCC 39 118, celles-ci étant récoltées, par exemple, par centrifugation ou filtration d'une culture dont la croissance a été effectuée dans un milieu approprié que l'on décrira ci-dessous.

Selon l'invention, l'utilisation de cellules entières, comparativement à l'emploi d'extraits enzymatiques bruts ou purifiés, s'accompagne de nombreux avantages parmi lesquels, on peut noter :

- la suppression des étapes de broyage des microorganismes, de centrifugation des broyats pour récupérer les extraits enzymatiques bruts et éventuellement de purification des extraits bruts, toutes ces opérations s'accompagnant en outre de pertes non négligeables au niveau des activités enzymatiques recherchées ;
- l'obtention, au cours de la réaction de transformation de l'α cétoacide en L-α aminoacide, d'un milieu réactionnel extracellulaire pas ou peu contaminé par les acides nucléiques et les protéines autres que les enzymes nécessaires à la bioconversion, toujours présents dans les extraits enzymatiques. Cela se traduit par une plus grande simplicité du processus de purification du produit recherché en fin de réaction.
- une meilleure stabilité au cours du temps des systèmes enzymatiques.

Selon une autre caractéristique de l'invention on peut utiliser les cellules entières qui ont subi au

préalable un traitement chimique ou physicochimique de perméabilisation. Les traitements de perméabilisation sont généralement effectués dans des conditions telles que les activités enzymatiques impliquées dans la bioconversion ne sont sensiblement pas altérées. Ils ont pour but de favoriser le transfert des substrats de la réaction ainsi que celui du coenzyme du milieu extracellulaire vers l'intérieur de la cellule d'une part, et le transfert des produits de la réaction du milieu intracellulaire vers l'extérieur de la cellule d'autre part.

Parmi les techniques de perméabilisation des microorganismes, bien connues de l'homme de l'art, on peut utiliser des méthodes chimiques qui consistent à mettre les bactéries en présence :

- de certains solvants tels que le toluène, l'acétone, le méthanol, le diméthylsulfoxyde, l'éther éthylique, l'alcool phénéthyle, etc...
- de certains détergents tels que le Brij 58®, le bromure de N cétyl -N,N,N triméthylammonium, le dodecylsulfate de sodium, le laurylsulfate de sodium, le triton X 100, le tween 80, etc...
- de certains agents chélatants tels que l'éthylène diamine tétracétate (EDTA).
- de certains antibiotiques tels que la nystatine, l'amphotéricine B, etc.

On peut encore employer des méthodes physiques comme le broyage (par sonication par exemple), la congélation, le séchage, la lyophilisation, le chauffage, etc.

Selon le procédé de l'invention, on peut utiliser des cellules entières sous forme immobilisées ou les cellules entières perméabilisées puis immobilisées.

Parmi les techniques d'immobilisation des microorganismes bien connues de l'homme de l'art, on peut utiliser :

- les techniques d'adsorption des microorganismes sur des support tels que du kieselguhr, de la brique pilée, des résines échangeuses d'ions, des billes de verre, des copeaux de bois, de la céramique, etc,
- les techniques d'inclusion des microorganismes dans des gels de polymères tels que l'agar, l'alginate de calcium, le carrageenane, le polyacrylamide, etc,
- les techniques de couplage des microorganismes sur différents supports, billes de silice, verres frittés, par liaison covalente avec des agents tels que du glutaraldéhyde, des isocyanates, etc.

Des conditions préférées pour la conduite de la culture de Bacillus, de Bacillus megaterium et de la souche ATCC 39118 sont les suivantes :

La présence dans le milieu, de mono- ou oligosaccharides tels que par exemple le glucose, le lactose, le fructose ou le saccharose à une concentration d'au moins 1 g/l et de manière préférée, de 1 à 40 g/litre s'est généralement avérée utile pour l'obtention d'activités glucose deshydrogénase et L-aminoacide deshydrogénase élevées. L'addition d'une source d'azote minéral (sous forme d'ions ammonium) et/ou d'azote organique et de manière préférée d'une quantité de 0,1 à 3% d'azote organique sous forme d'urée par exemple ou sous forme d'aminoacides telle que notamment l'extrait de levure, la peptone de soja, des hydrolysats de caséine, la liqueur de macération du maïs, des extraits de viande peuvent permettre d'obtenir en milieu aérobie une bonne croissance et des activités enzymatiques élevées. L'addition d'éléments minéraux tels que par exemple le potassium, le sodium, le magnesium et d'oligoéléments tels que par exemple le fer, le manganèse, le molybdène sous forme de leurs sels (sulfates, phosphates, chlorures) peuvent permettre également d'améliorer encore la croissance. Les conditions de milieu de culture sont habituellement les suivantes : température : 20 à 40 °C, pH = 5,5 à 8,0. Un excellent niveau d'activité a été obtenu à une température comprise entre 27 et 32°C et dans un intervalle de pH de 6,5 à 7,5.

A partir des cultures de Bacillus, ou de Bacillus megaterium et en particulier de la souche ATCC 39118, on récolte, par exemple, par centrifugation, les bactéries. On mesure de manière conventionnelle à partir d'un extrait d'une partie aliquote de ces bactéries les activités enzymatiques dans les conditions suivantes : T° : 30°C, pH d'activité maximum pour chaque enzyme et concentration de substrat saturante.

Puisqu'il est régénéré, au lieu d'être utilisé dans des proportions sensiblement stoechiométriques, le coenzyme sous forme réduite (NADH) ou oxydée ($NAD^+$) peut être utilisé à une concentration de 0,1 à 10 mmoles par litre et de manière préférée de 0,3 à 3 mmoles par litre. Il peut notamment être mis en oeuvre sous une forme polymérique à haut poids moléculaire (500 - 50 000). On a obtenu de très bons résultats de production de L-$\alpha$ aminoacides lorsque le réducteur du coenzyme était le glucose.

La synthèse proprement dite des L-$\alpha$ aminoacides s'effectue dans un réacteur maintenu à une température de 10 à 60°C, de préférence de 20 à 45°C. Le pH du milieu est maintenu à une valeur choisie entre 6 et 10. On le maintient de préférence sensiblement constant par addition d'une base, de préférence l'ammoniaque. L'addition d'ammoniaque dans le réacteur est doublement nécessaire, à la fois comme agent de régulation mais aussi comme réactif puisqu'il intervient dans la première réaction enzymatique, à savoir l'amination réductrice de l'$\alpha$-cétoacide. On a obtenu de bons résultats en injectant de 1 à 3 moles exprimées en ammoniaque, de la source d'ion ammonium pour 1 mole d'$\alpha$-cétoacide.

Un pH situé entre 6,5 et 8,5 est particulièrement favorable pour la stabilité des enzymes utilisées et du nicotinamide adénine dinucléotide. Le choix du pH est en effet important pour ce dernier composé dont la forme réduite est instable en milieu acide et la forme oxydée instable en milieu alcalin. La présence dans le milieu d'un tampon, par exemple un sel soluble de l'acide phosphorique tel qu'un phosphate d'ammonium, peut être utile pour maintenir le pH du milieu et la stabilité des enzymes. On peut encore ajouter des agents de stabilisation des enzymes parmi lesquels les composés comportant un groupement thiol, tels que le 2-mercaptoéthanol, le dithiothréitol, et..., sont particulièrement efficaces.

Un mode opératoire préféré est décret ci-après : on introduit dans le réacteur les éléments choisis, notamment le tampon, le $NAD^+$ (ou le NADH) et soit l'extrait enzymatique préparé à partir d'une culture de Bacillus, de Bacillus megaterium ou de la souche ATCC 39118, soit les bactéries entières récoltées après centrifugation de la culture qui ont été perméabilisées, et/ou immobilisées, ou non traitées au préalable. Une partie seulement du réducteur, par exemple le glucose, nécessaire à la réaction est introduite en début de réaction. En effet, on préfère éviter la présence dans le réacteur de concentrations trop fortes en glucose qui peuvent avoir une action inhibitrice sur l'activité enzymatique L-aminoacide deshydrogénase. De la même façon, on préfère éviter la présence dans le réacteur de concentrations élevées en α-cétoacides qui peuvent avoir une action inhibitrice sur l'activité enzymatique L-aminoacide deshydrogénase. C'est pourquoi, il est souvent avantageux d'injecter en continu dans le réacteur ces deux composés sous forme d'une solution aqueuse dont la quantité de réducteur (glucose par exemple) est de 1 à 2 moles pour 1 mole de α-cétoacide. L'α-cétoacide peut être introduit sous forme d'acide ou de l'un de ses sels, notamment un sel de $Na^+$, $K^+$, $NH_4^+$ ,$Li^+$ Le débit d'injection est de préférence réglé afin que le paramètre limitant de la réaction soit l'apport de l'α-cétoacide, ce dernier composé étant alors transformé en L-aminoacide sensiblement au fur et à mesure de son introduction.

Le L-α-aminoacide formé au cours de la réaction peut être dosé par des techniques connues de l'homme de l'art, par exemple enzymatiquement au moyen de la L-aminoacide oxydase ou au moyen d'un analyseur automatique d'aminoacides.

En fin de réaction, on peut extraire le L-α aminoacide formé par exemple par passage sur une résine échangeuse de cations et ensuite évaporer l'éluat sous vide.

A titre d'exemple, le procédé selon l'invention permet de réaliser les synthèses suivantes à partir des α-cetoacides suivants ou de l'un de leurs sels :

- la L-alanine à partir d'acide pyruvique,
- la L-serine à partir de l'acide hydroxypyruvique
- la L-leucine à partir de l'acide α-cétoisocaproïque
- la L-valine à partir de l'acide 3-méthyl-2-oxobutyrique,
- la L-isoleucine à partir de l'acide 3-méthyl-2-oxovalérique et
- la L-phenylalanine à partir de l'acide phénylpyruvique.

Les exemples suivants qui illustrent l'invention sont donnés à titre non limitatif.

Exemple 1 : Préparation de la L-leucine

La souche de Bacillus megaterium ATCC 39118 est cultivée en fioles de Fernbach dans un milieu dont la composition est la suivante :

| | |
|---|---|
| - hydrolysat trypsique de caséine | 17 g |
| - peptone papaïnique de soja | 3 g |
| - chlorure de sodium | 5 g |
| - phosphate bipotassique | 2,5 g |
| - glucose | 2,5 g |
| - Eau distillée | 1 litre |

Ce milieu est stérilisé, avant ensemencement, 30 minutes à 115 ° C. La culture est réalisée à 30 ° C, pH = 7,0, pendant 24 heures. Ensuite les cellules sont récoltées par centrifugation, mises en suspension dans un tampon phosphate de potassium 100 mM, pH = 7,5, et cassées au cours d'un traitement aux ultra-sons pendant 2 mn en réfrigérant en permanence la suspension cellulaire de façon que sa température n'excède jamais 10 ° C. On élimine par centrifugation les débris cellulaires et on récupère l'extrait enzymatique sur lequel on détermine l'activité enzymatique de la glucose deshydrogénase et de la leucine deshydrogénase. L'activité enzymatique de la glucose deshydrogénase est trouvée égale à 0,23 unité par mg de protéines de l'extrait, celle de la leucine deshydrogénase est de 0,13 unité par mg de protéines de l'extrait. Une unité

enzymatique est définie comme la quantité d'enzyme permettant la transformation d'une micromole de substrat (le glucose dans le cas de la glucose deshydrogénase, l'acide α-cétoisocaproïque ou un de ses sels dans le cas de la leucine deshydrogénase) par minute dans les conditions de l'essai.

Une partie aliquote de cet extrait enzymatique a été concentrée environ 5 fois par ultrafiltration à + 4 ° C, et l'extrait enzymatique concentré obtenu a été employé ci-après pour la synthèse de la L-leucine.

La synthèse de la L-leucine est effectuée dans un réacteur de 150 ml, thermostaté à 30 ° C, avec un volume liquide initial de 50 ml, agité par l'intermédiaire d'un barreau magnétique. Le milieu liquide initial renferme les composants suivants :

- tampon phosphate d'ammonium pH = 8,2 1 mole/l
- $NAD^+$ 1 mmole/l
- glucose 50 mmoles/l
- extrait enzymatique concentré tel qu'obtenu ci-dessus = 12,5 ml soit 290 unités de glucose deshydrogénase et 122 unités de leucine deshydrogénase

On injecte en continu une solution à pH = 7,0, contenant 200 mmoles par litre de cétoisocaproate de sodium et 220 mmoles par litre de glucose, à un débit de 1,5 ml/h. Le pH est maintenu à 8,2 par addition d'ammoniaque 2N.

Après 24 heures, le volume liquide dans le réacteur est de 86 ml, et la concentration en L-leucine formée, mesurée par le dosage enzymatique à la L-aminoacide oxydase, est de 65 mmoles par litre. La production en L-leucine est égale à 5,6 mmoles avec un rendement molaire de 78 % par rapport à l'α-cétoisocaproate de sodium injecté. La consommation d'ammoniaque 2N est de 5,7 millilitres.

La purification des L-α aminoacides décrits dans les exemples s'effectue de la manière suivante :
Le milieu réactionnel est versé en haut d'une colonne de résine échangeuse de cations Amberlite® ER 151 préalablement mise sous forme $H^+$ par lavage avec de l'acide chlorhydrique 1N suivi d'un lavage à l'eau. Après introduction de l'échantillon, la colonne est à nouveau lavée par de l'eau puis éluée avec de l'ammoniaque 2N. Après le passage d'un volume de la solution d'élution correspondant au volume "mort" de la colonne, on commence à récupérer les fractions éluées jusqu'à ce que leur volume total représente 1,5 fois le volume mort de la colonne. L'éluat est ensuite évaporé sous vide, redissous dans l'eau et à nouveau évaporé. Le rendement de la purification est de 98 %.

Exemple 2 : Préparation de L-valine

La préparation de l'extrait enzymatique est effectuée dans les mêmes conditions que celles décrites dans l'exemple 1, sauf que la culture de Bacillus megaterium ATCC 39118 dure 30 heures et que l'extrait enzymatique est utilisé à l'état brut. L'activité enzymatique de la glucose deshydrogénase et de la L-valine deshydrogénase sont trouvées chacune égales à 0,3 unité par mg de protéines de l'extrait.

La synthèse de la L-valine est réalisée dans un réacteur de 150 ml, agité, et thermostaté à 30 ° C. Les composants du milieu dont le volume initial est de 50 ml, sont les mêmes que dans l'exemple 1, en présence d'un extrait enzymatique de 8,5 ml contenant 50 unités de glucose deshydrogénase et 50 unités de L-valine deshydrogénase
On injecte en continu une solution à pH = 7,0 contenant 200 mmoles par litre de 3-méthyl-2-oxobutyrate de sodium et 220 mmoles par litre de glucose, à un débit de 1,5 ml/h. Le pH est maintenu constant à 8,2 par addition d'ammoniaque 2N.

La réaction de synthèse est arrêtée après 24 heures. Le volume liquide dans le réacteur est alors de 90 ml et la concentration en L-valine formée, est égale à 80 mmoles par litre. Le rendement molaire de la synthèse de L-valine est de 100 % par rapport au 3-méthyl-2-oxobutyrate de sodium introduit dans le réacteur, et la production en L-valine égale à 7,2 mmoles. La consommation en ammoniaque 2N est de 7,4 millilitres.

Exemple 3 : Préparation de L-valine par des cellules de Bacillus megaterium ATCC 39 118 sans ajout de coenzyme

La synthèse de L-valine est effectuée dans les mêmes conditions que celles décrites dans l'exemple 2, sauf que l'on ne rajoute pas de $NAD^+$ dans le milieu initial.

Après 24 heures de réaction, la concentration en L-valine est toujours égale à 0 et le 3-méthyl-2-oxobutyrate de sodium n'a pas été consommé.

Exemple 4 : Préparation de L-valine par un extrait brut de Bacillus subtilis 168 M.

La souche de Bacillus subtilis 168 M est cultivée dans les mêmes conditions que celles de l'exemple 1 pendant 30 heures. L'activité glucose déshydrogènase de l'extrait brut issu de cette culture est de 0,07 unité par mg de protéine et l'activité L-valine-déshydrogénase mesurée en présence de 3-méthyl-2-oxobutyrate est de de 0,08 unité par mg de protéines.

Après concentration, par exemple, par ultrafiltration de l'extrait enzymatique, on réalise à partir du 3-méthyl-2-oxobutyrate de sodium la synthèse de la L-valine selon l'exemple 2 et on obtient un rendement de 18%.

Exemple 5 : Préparation de L-isoleucine

On emploie le même extrait enzymatique que celui décrit dans l'exemple 2. L'activité enzymatique de la glucose deshydrogénase est égale à 0,30 unité par mg de protéines de l'extrait, celle de la L-isoleucine deshydrogénase 0,25 unité par mg de protéines de l'extrait.

On réalise la synthèse de la L-isoleucine dans l'appareillage déjà décrit dans les exemples précédents. Les composants du milieu, dont le volume initial est 50 ml, sont les mêmes que dans l'exemple 1, en présence d'un extrait enzymatique de 9 ml contenant 53 unités de glucose deshydrogénase et 44 unités de L-isoleucine deshydrogénase.

On injecte en continu une solution à pH = 7,0, contenant 200 mmoles par litre de 3-méthyl-2-oxovalérate de sodium et 220 mmoles par litre de glucose, à un débit de 1,5 ml/h. Le pH est maintenu à 8,2 par addition d'ammoniaque 2N.

Après 24 heures de réaction, le volume liquide dans le réacteur est de 89 ml et la concentration en L-isoleucine formée, est égale à 78 mmoles par titre. Le rendement molaire de synthèse de la L-isoleucine par rapport au 3-méthyl-2-oxovalérate de sodium injecté est de 97 %.

Exemple 6 : Préparation de la L-phénylalanine

L'extrait enzymatique est préparé comme dans l'exemple 2 et on détermine une activité enzymatique L-phénylalanine deshydrogénase de 0,03 unité par mg de protéines et une activité glucose deshydrogénase de 0,30 unité par mg de protéines.

Après concentration de l'extrait, on réalise la préparation de la L-phénylalanine à partir du phénylpyruvate de sodium, selon l'exemple 1 et on obtient un rendement après purification de 38 %.

Exemple 7 : Préparation du L-valine par extrait brut de Bacillus megaterium ATCC 39 118 immobilisé.

La préparation de l'extrait enzymatique est effectuée dans des conditions similaires à celles utilisées dans l'exemple 1, à l'exception du temps de culture de Bacillus megaterium ATCC 39 118, qui a été allongé de 30 à 40 heures.

L'activité enzymatique de la glucose déshydrogènase est trouvée égale à 0,93 unité par mg de protéines et l'activité enzymatique de la L-valine déshydrogénase est de 0,28 unité par mg de protéines.

Une partie de l'extrait cellulaire est soumis à un traitement d'immobilisation au sein de billes d'alginate de calcium. Pour cela, de l'alginate de sodium est ajouté à une solution de chlorure de sodium à 0,9% , et 16ml de cette solution sont mélangés à 8ml d'extrait. La solution d'alginate est preparée afin d'obtenir une concentration finale d'alginate de 2% dans le mélange avec l'extrait cellulaire.

Le mélange alginate extrait est versé régulièrement goutte à goutte dans une solution de $CaCL_2$ 0,1 M agitée. Quand toutes les billes sont formées, on les laisse en contact avec la solution de $CaCl_2$ pendant 2 heures sous agitation. Les billes de gel d'alginate de calcium renfermant l'extrait sont ensuite récupérées par filtration puis lavées avec une solution de NaCl à 0,9%. Les billes de gel peuvent être conservées à 4 ° C dans un tampon succinnate 0,1 M pH5 contenant du $CaCl_2$ 10 millimolaire.

La synthèse de la L-valine est effectuée dans des conditions initiales identiques à celles de l'exemple 2, sauf que le réacteur contient 8 ml d'extrait immobilisé dans l'alginate, soit 276 unités de glucose déshydrogénase et 84 unités de L-valine déshydrogénase.

On injecte en continu une solution à pH 7,0 contenant 300 mmoles par litre de 3-méthyl-2-oxobutyrate de sodium et 330 mmoles par litre de glucose, à un débit de 0,5 ml/h. Le pH est maintenu constant à 8,2 par addition d'ammoniaque 2N.

La réaction de synthèse est arrêtée après 25 heures. Le volume réactionnel final est de 64 ml et la concentration en valine formée est égale à 40 mmoles par litre. Le rendement molaire de la syntèse de L-valine par rapport au 3-méthyl-2-oxobutyrate de sodium injecté est de 69%, et la production de L-valine égale à 2,6 mmoles. La consommation en ammoniaque 2 N est de 6,3 ml.

Exemple 8 : Préparation de L-alanine par un extrait brut de Bacillus megaterium ATCC 39 118 cultivé sur un milieu à base d'extrait de levure.

La souche de Bacillus megaterium ATCC 39 118 est cultivée dans un milieu dont la composition est la suivante :

| | |
|---|---|
| - extrait de levure | 20 g |
| - lactose | 3 g |
| - sulfate de magnésium | 0,5 g |
| - phosphate monopotassique | 2 g |
| - chlorure de manganèse | 40 mg |
| - sulfate de fer | 2,5 mg |
| - molybdate d'ammonium | 2 mg |
| - eau de ville | 1 l |

Le pH du milieu est ajusté à 7,3 avant stérilisation. Un volume de 300 ml de milieu cultivé pendant 24 heures sert à ensemencer un fermenteur contenant 3,7 litres de ce même milieu. La fermentation a lieu à 30° C, la vitesse d'agitation est de 250 tours/minute, l'aération est de 0,5 vvm et le pH n'est pas régulé. La teneur en oxygène dissous descend à 0% après 2 heures de culture. Après 18 heures de fermentation, celle-ci est arrêtée et les cellules sont récoltées par centrifugation en continu avant cassage aux ultrasons pour l'obtention des extraits bruts. L'activité alanine-déshydrogénase mesurée en présence d'acide pyruvique est égale à 1,48 unités par mg de protéines et l'activité glucose déshydrogénase est de 0,42 unité par mg de protéines.

La synthèse de L-alanine est effectuée dans les conditions de l'exemple 1, exception faite du volume initial de milieu qui est de 57 ml, de la quantité d'extrait enzymatique ajouté (24,7ml) qui correspond à 132 unités de glucose déshydrogènase et 464 unités d'alanine déshydrogénase, et du précurseur injecté qui est du pyruvate de sodium.

Après 43 heures de réaction, la concentration en L-alanine est de 63 mmoles par litre. Le rendement molaire de production de L-alanine par rapport au pyruvate de sodium injecté est de 79%. En présence d'un milieu de culture tel que dans l'exemple 1, l'activité L-alanine déshydrogénase dans l'extrait brut est de 0,04 unités par mg de protéines pour une activité glucose déshydrogénase de 0,3 unité par mg de protéines. Après concentration de l'extrait enzymatique, en procèdant comme dans l'exemple 1, on obtient un rendement de 52% en L-alanine à partir de pyruvate de sodium.

Exemple 9 : Préparation de L-sérine par un extrait brut de Bacillus megaterium ATCC 39118 cultivé sur un milieu à base d'extrait de levure

On utilise le même extrait enzymatique que celui obtenu dans l'exemple 8. L'activité glucose déshydrogénase de l'extrait est donc inchangée et l'activité sérine déshydrogénase mesurée en présence d'hydroxypyruvate de lithium en tant que précurseur est de 0,63 unité par mg de protéine.

La synthèse de L-sérine est effectuée dans des conditions identiques à celles décrites dans l'exemple 8 exception faite du nombre d'unités sérine déshydrogénase initialement présentes dans le réacteur qui est de 197 unités et du précurseur injecté qui est remplacé par l'hydroxypyruvate de lithium.

Après 44 heures de réaction, la concentration en L-sérine dans le réacteur est de 45 mmoles par litre et le rendement molaire de production de L-sérine formée par rapport à l'hydroxypyruvate de sodium injecté est de 56 %.

Exemple 10 : Préparation de L-valine par des cellules de Bacillus megaterium ATCC 39 118.

La souche de Bacillus megaterium ATCC 39 118 est cultivée dans les mêmes conditions que celles décrites dans l'exemple 1, pendant 24 heures, puis les cellules sont récoltées par centrifugation. Le culot bactérien est réparti en quatre fractions qui seront utilisées dans quatre essais différents de synthèse de L-valine effectués dans les conditions de l'exemple 2, sauf que

- dans le premier essai, les cellules récoltées après centrifugation ne subissent aucun traitement avant d'être introduites dans le réacteur. La quantité de cellules utilisées correspond à un poids sec de 0,145 g.
- dans le second essai, les cellules récoltées après centrifugation (5,5g de culot bactérien, soit 1,16g de

poids sec de cellules) sont immobilisées dans des billes de gel d'alginate en procédant de la même manière que celle décrite dans l'exemple 7, avant d'être introduites dans le réacteur.

- dans le troisième essai, les cellules récoltées par centrifugation sont soumises à un traitement de perméabilisation à l'acétone avant d'être introduites dans le réacteur. Le culot bactérien, correspondant à un poids sec de 0,16g est mis en suspension dans 5ml de tampon phosphate 100 mM, pH = 7,5, contenant 20% d'acétone, 5% de glucose et 0,04% d'azide de sodium. L'ensemble est mis en agitation pendant 20 minutes avant qu'on y ajoute 5 ml du même tampon sans acétone. Les cellules perméabilisées sont récoltées par centrifugation et remises en suspension dans 5 ml de tampon sans acétone avant d'être introduites dans le réacteur.
- dans le quatrième essai, les cellules récoltées par centrifugation sont mises en suspension dans un tampon phosphate 0,1M pH 7,5, et traitées aux ultra-sons pendant 2 minutes en réfrigérant la suspension. Le broyat récupéré est ensuite immobilisé dans un gel d'alginate en procédant de la même manière que celle décrite dans l'exemple 7, sauf que l'on remplace les 8ml d'extrait enzymatique par 8ml de broyat, avant d'être introduit dans le réacteur.

Les résultats de la synthèse de la L-valine à partir de 3-méthyl-2-oxobutyrate, pour ces quatre essais sont regroupés dans le tableau suivant :

| Nature de l'essai | 1er essai | 2ème essai | | 3ème essai | 4ème essai | |
|---|---|---|---|---|---|---|
| | Cellules entières | Cellules entières immobilisées | | Cellules perméabilisées | Cellules perméabilisees et immobilisées | |
| Temps de réaction (h) | 23 | 24 | 100 | 23 | 20 | 96 |
| Rendement molaire de synthèse de la L-valine (%) | 89 | 65 | 79 | 80 | 100 | 90 |

## Revendications

1. Procédé de production de L-$\alpha$ aminoacide, dans lequel on soumet un mélange d'au moins un $\alpha$-cétoacide ou d'au moins l'un de ses sels, d'une source d'ions ammonium, de nicotinamide adénine dinucléotide sous forme réduite ou oxydée et d'un réducteur à l'action d'une composition enzymatique produite par culture d'une souche de microorganisme, caractérisé en ce qu'on utilise une souche de Bacillus comme seule souche de microorganisme.

2. Procédé selon la revendication 1, dans lequel la souche est celle de Bacillus megaterium.

3. Procédé selon l'une quelconque des revendications 1 ou 2, dans lequel la souche est celle de Bacillus megaterium ATCC 39118.

4. Procédé selon l'une quelconque des revendications 1 à 3, dans lequel on utilise l'extrait brut ou purifié de ladite souche de microorganisme.

5. Procédé selon l'une quelconque des revendications 1 à 3, dans lequel on utilise les cellules entières de ladite souche de microorganisme.

6. Procédé selon la revendication 4, dans lequel ledit extrait est immobilisé.

7. Procédé selon la revendication 5, dans lequel on utilise les cellules entières perméabilisées, les cellules entières immobilisées, ou les cellules entières perméabilisées puis immobilisées.

8. Procédé selon l'une quelconque des revendications 1 à 7, dans lequel ledit produit de culture a été obtenu en présence de mono ou oligosaccharides à la concentration de 1 à 40 g/litre.

9. Procédé selon l'une quelconque des revendications 1 à 8, dans lequel le produit de culture a été obtenu par culture de Bacillus à une température de 20 à 40 °C et à un pH de 5,5 à 8 dans un milieu de culture renfermant au moins une source d'azote minéral et/ou d'azote organique.

10. Procédé selon l'une quelconque des revendications 1 à 9, dans lequel ladite production de L-α aminoacide est mise en oeuvre à une température de 10 à 60 °C et à un pH de 6 à 10.

11. Procédé selon l'une quelconque des revendications 1 à 10, dans lequel le réducteur est le glucose.

12. Procédé selon l'une quelconque des revendications 1 à 11, dans lequel, dans une opération continue, pour 1 mole d'α cétoacide, on met en oeuvre de 1 à 2 moles de réducteur et de 1 à 3 moles, exprimées en ammoniaque, de la source d'ions ammonium.

13. Procédé selon l'une quelconque des revendications 1 à 12, dans lequel on met en oeuvre le procédé avec une composition enzymatique présentant une activité L- aminoacide deshydrogénase et une activité glucose deshydrogénase en proportions de 0,1 : 1 à 10 : 1.

14. Procédé selon l'une quelconque des revendications 1 à 13, dans lequel l'α-cétoacide est l'acide pyruvique, l'acide hydroxypyruvique, l'acide α-cétoisocaproïque, l'acide 3-méthyl-2-oxobutyrique, l'acide 3-méthyl-2-oxovalérique, l'acide phénylpyruvique ou l'un de leurs sels.

## Claims

1. A process for producing L-α aminoacid, wherein a mixture of at least one α -ketoacid or at least one salt thereof, a source of ammonium ions, nicotinamide adenine dinucleotide in reduced or oxidized form and a reducing agent is subjected to the action of an enzyme composition produced by culture of a microorganism strain, characterized by the use of a Bacillus strain as the only microorganism strain.

2. A process according to claim 1, wherein the strain is that of Bacillus megaterium.

3. A process according to any one of claims 1 or 2, wherein said strain is that of Bacillus megaterium ATCC 39 118.

4. A process according to any one of claims 1 to 3, wherein the raw or purified extract of said microorganism strain is used.

5. A process according to any one of claims 1 to 3, wherein whole cells of said microorganism strain are used.

6. A process according to claim 4, wherein said extract is fixed.

7. A process according to claim 5, using permeabilized whole cells, fixed whole cells, or permeabilized and then fixed whole cells.

8. A process according to any one of claims 1 to 7, wherein said culture product has been obtained in the presence of mono- or oligo-saccharides at a concentration of 1 to 40 g/liter.

9. A process according to any one of claims 1 to 8, wherein the culture product has been obtained by culture of Bacillus at a temperature from 20 to 40 ° C and at pH of 5.5 to 8 in a culture medium containing at least one source of inorganic nitrogen and/or organic nitrogen.

10. A process according to any one of claims 1 to 9, wherein said production of L-α aminoacid is performed at a temperature from 10 to 60 ° C and at a pH from 6 to 10.

11. A process according to any one of claims 1 to 10 wherein the reducing agent is glucose.

12. A process according to any one of claims 1 to 11, wherein, in a continuous operation, 1 to 2 moles of reducing agent and 1 to 3 moles, expressed as ammonia, of the ammonium ions source, are used per mole of α -ketoacid.

13. A process according to any one of claims 1 to 12, wherein the enzyme composition used in the process has a L -aminoacid dehydrogenase activity and a glucose dehydrogenase activity in proportions of 0.1 : 1 to 10 : 1.

14. A process according to any one of claims 1 to 13, wherein the α -ketoacid is pyruvic acid, hydroxypyruvic acid, α -ketoisocaproic acid, 3-methyl-2-oxobutyric acid, 3-methyl-2-oxovaleric acid, phenyl-pyruvic acid or one of their salts.

**Patentansprüche**

1. Verfahren zur Produktion von L-α-Aminosäure, bei dem eine Mischung von mindestens einer α-Ketosäure oder mindetens einem ihrer Salze, einer Ammoniumionenquelle, Nikotinsäureamidadenindinucleotid in reduzierter oder oxidierter Form und einem Reduktionsmittel dem Einwirken einer enzymatischen Zusammensetzung unterworfen wird, die von einer Kultur eines Stammes eines Mikroorganismus gebildet ist,
dadurch gekennzeichnet,
daß ein Stamm des Bacillus als einziger Stamm eines Mikroorganismus verwendet wird.

2. Verfahren nach Anspruch 1,
bei dem der Stamm der des Bacillus megaterium verwendet wird.

3. Verfahren nach einem der Ansprüche 1 oder 2,
bei dem der Stamm der des Bacillus megaterium ATCC 39 118 verwendet wird.

4. Verfahren nach einem der Ansprüche 1 bis 3,
bei dem der Rohauszug oder der gereinigte Auszug des Stammes des Mikroorganismus verwendet wird.

5. Verfahren nach einem der Ansprüche 1 bis 3,
bei dem die ganzen Zellen des Stammes des Mikroorganismus verwendet werden.

6. Verfahren nach Anspruch 4,
bei dem der Auszug immobilisiert ist.

7. Verfahren nach Anspruch 5,
bei dem die ganzen permeabilisierten Zellen, die ganzen immobilisierten Zellen oder die ganzen permeabilisierten und dann immobilisierten Zellen verwendet werden.

8. Verfahren nach einem der Ansprüche 1 bis 7,
bei dem das Produkt der Kultur in Anwesenheit von Mono- oder Oligosacchariden einer Konzentration von 1 bis 40 g/l erhalten wurde.

9. Verfahren nach einem der Ansprüche 1 bis 8,
bei dem das Produkt der Kultur durch kultivieren des Bacillus bei einer Temperatur zwischen 20 und 40 ° C und einem pH-Wert zwischen 5,5 und 8 in einem Milieu einer Kultur erhalten wird, die mindestens eine Quelle von anorganischem Stickstoff und/oder von organischem Stickstoff beinhaltet.

10. Verfahren nach einem der Ansprüche 1 bis 9,
bei dem die Produktion der L-α-Aminosäure bei einer Temperatur zwischen 10 und 60 ° C und einem pH-Wert zwischen 6 und 10 durchgeführt wird.

11. Verfahren nach einem der Ansprüche 1 bis 10,
bei dem das Reduktionsmittel Glukose ist.

12. Verfahren nach einem der Ansprüche 1 bis 11,
bei dem für ein Mol der α-Ketosäure in einem kontinuierlichen Vorgehen 1 bis 2 Mol des Reduktionsmittels und 1 bis 3 Mol der Ammoniumionenquelle, ausgedrückt in Ammoniak, hinzugefügt werden.

13. Verfahren nach einem der Ansprüche 1 bis 12,
wobei das Verfahren mit einer enzymatischen Zusammensetzung durchgeführt wird, die eine L-α-Aminosäuredehydrogenaseaktivität und eine Glukosedehydrogenaseaktivität in einem Verhältnis zwischen 0,1:1 und 10:1 aufweist.

14. Verfahren nach einem der Ansprüche 1 bis 13,
bei dem die α-Ketosäure Brenztraubensäure, Hydroxy-Brenztraubensäure, α-Ketoisokapronsäure, 3-Methyl-2-Oxobuttersäure, 3-Methyl-2-Oxovaleriansäure, Phenyl-Brenztraubensäure ist oder eines deren Salze.